# EUROPÄISCHE PATENTSCHRIFT

(11) **EP 3 092 052 B1**
(45) Veröffentlichungstag und Bekanntmachung des Hinweises auf die Patenterteilung: **01.11.2017**
(21) Anmeldenummer: 14815335.6
(22) Anmeldetag: 17.12.2014
(51) Int. Cl.: B01D 53/14, B01D 3/14, C07C 7/04, C07C 7/09, C07C 7/11, C10G 70/04, C10G 70/06, F25J 3/02

(54) **VERFAHREN ZUR TRENNUNG EINES WASSERSTOFFHALTIGEN KOHLENWASSERSTOFFGEMISCHS, TRENNEINRICHTUNG UND OLEFINANLAGE**
METHOD FOR SEPARATING A MIXTURE CONTAINING HYDROGEN AND HYDROCARBONS, SEPARATING DEVICE AND OLEFIN PLANT
PROCÉDÉ DE SÉPARATION D'UN MÉLANGE D'HYDROCARBURES CONTENANT DE L'HYDROGÈNE, DISPOSITIF DE SÉPARATION ET INSTALLATION D'OLÉFINE

(30) Priorität: 07.01.2014 EP 14000040
(43) Veröffentlichungstag der Anmeldung: 16.11.2016
(73) Patentinhaber: Linde AG, 80331 Munich (DE)
(72) Erfinder: FRITZ, Helmut, 81375 München (DE); HÖFEL, Torben, 81543 München (DE)
(86) Internationale Anmeldenummer: PCT/EP2014/078168
(87) Internationale Veröffentlichungsnummer: WO 2015/104153

(56) Entgegenhaltungen:
- DE-A1-102005 003 499
- DE-A1-102005 047 342
- DE-A1-102005 050 388
- US-A- 5 685 170
- US-A1- 2007 225 537

## Beschreibung

Die Erfindung betrifft ein Verfahren zur Trennung eines Wasserstoff enthaltenden Kohlenwasserstoffgemischs unter Gewinnung eines wasserstoffreichen Stroms, eine entsprechende Trenneinrichtung sowie eine Olefinanlage mit einer entsprechenden Trenneinrichtung gemäß den Oberbegriffen der unabhängigen Patentansprüche.

### Stand der Technik

Verfahren und Vorrichtungen zum Dampfspalten (engl. Steam Cracking) von Kohlenwasserstoffen sind bekannt und beispielsweise im Artikel "Ethylene" in Ullmann's Encyclopedia of Industrial Chemistry, online seit 15. April 2007, DOI 10.1002/14356007.a10_045.pub2, beschrieben.

Durch Dampfspalten, aber auch unter Verwendung anderer Verfahren und Vorrichtungen, werden Kohlenwasserstoffgemische erhalten, die zumindest teilweise in die jeweils enthaltenen Komponenten aufgetrennt werden müssen. Dies kann auf unterschiedliche Weise erfolgen.

Die vorliegende Erfindung geht beispielsweise von einer Trennung aus, in der ein Kohlenwasserstoffstrom anfällt, der im Wesentlichen aus Kohlenwasserstoffen mit zwei Kohlenstoffatomen, Methan und Wasserstoff besteht. Wie unten erläutert, wird ein derartiger Strom auch als "C2minus-Strom" bezeichnet. Die Erfindung eignet sich jedoch auch für andere Trennungen, wie unten erläutert.

Herkömmliche Verfahren umfassen, einen derartigen C2minus-Strom unter Druck stufenweise in Wärmetauschern abzukühlen und stromab dieser Wärmetauscher jeweils ein flüssiges Kondensat abzuscheiden. Ein bei einem Druck von ca. 35 bar abs. und bei einer Temperatur von unter ca. -100 °C gasf örmig verbleibender Anteil wird dabei typischerweise in einen sogenannten C2-Absorber (auch kurz als "Absorber" bezeichnet) eingespeist. Dieser weist beispielsweise 14 Böden auf und wird bei ca. 35 bar und unterhalb von -100 °C betrieben.

Am Kopf des C2-Absorbers wird ein flüssiger Rücklauf aufgegeben. Hierbei handelt es sich um im Wesentlichen reines Methan. Dieses stammt in herkömmlichen Verfahren aus einer Destillationssäule (sogenannter Demethanizer), in die die aus dem C2minus-Strom abgeschiedenen Kondensate sowie ein Kondensat, das im Sumpf des C2-Absorbers selbst abgeschieden wird, eingespeist werden.

Im Sumpf der Destillationssäule wird ein Kondensat, das im Wesentlichen aus Kohlenwasserstoffen mit zwei Kohlenwasserstoffen besteht, abgeschieden. Ein gasförmig verbleibender Anteil (im Wesentlichen reines Methan) wird am Kopf der Destillationssäule abgezogen und in einem Kopfkondensator gegen ein Kältemittel (teilweise) verflüssigt. Ein Teil des Verflüssigungsprodukts wird als der erwähnte flüssige Rücklauf auf den C2-Absorber aufgegeben.

Da die Destillationssäule bei einem geringeren Druck betrieben wird als der C2-Absorber, nämlich bei ca. 28 bis 34 oder 30 bis 32 bar abs., muss der flüssige Rücklauf vor dem Einspeisen in den C2-Absorber herkömmlicherweise mittels einer Pumpe auf Druck gebracht werden.

Neben Olefinen und anderen Kohlenwasserstoffen ist auch Wasserstoff ein wichtiges Produkt entsprechender Anlagen, da er beispielsweise für Hydrierzwecke (z.B. zur Hydrierung von Acetylenen oder in Hydrotreatmentverfahren in Erdölraffinerien) eingesetzt werden kann. Wasserstoff kann auch exportiert werden und ist dann ein wirtschaftlich mindestens so attraktives Produkt wie Ethylen.

Aus der DE 10 2005 003 499 A1 ist ein Verfahren bekannt, bei dem ein C2-Absorber, zwei Destillationssäulen und ein Rücklaufbehälter in Form einer einzigen Kolonne ausgebildet sind und bei einem nahezu einheitlichen Druckniveau betrieben werden.

Ein Verfahren zur Verarbeitung eines durch Dampfspalten erhaltenen Kohlenwasserstoffgemischs ist auch aus der DE 10 2005 050 388 A1 bekannt, aus der US 5 685 170 A ein Verfahren zur Rückgewinnung von Propan aus Erdgas.

Der Betrieb von Pumpen und Expandern für tiefkalte Medien ist schwierig und erfordert erhöhte Aufmerksamkeit. Es besteht daher der Bedarf nach Verbesserungen in entsprechenden Verfahren, wobei jedoch weiterhin ein wasserstoffreicher Strom gewonnen werden soll.

### Offenbarung der Erfindung

Vor diesem Hintergrund schlägt die Erfindung ein Verfahren zur Trennung eines Wasserstoff enthaltenden Kohlenwasserstoffgemischs, eine entsprechende Trenneinrichtung sowie eine Olefinanlage mit einer entsprechenden Trenneinrichtung mit den jeweiligen Merkmalen der unabhängigen Patentansprüche vor. Bevorzugte Ausgestaltungen sind Gegenstand der abhängigen Patentansprüche und der nachfolgenden Beschreibung.

Vor der Erläuterung der Merkmale und Vorteile der vorliegenden Erfindung werden deren Grundlagen und die verwendeten Begriffe erläutert.

Die vorliegende Erfindung kommt insbesondere zur Trennung von wasserstoffhaltigen Kohlenwasserstoffgemischen zum Einsatz, die durch Dampfspaltverfahren erhalten werden, ist jedoch nicht hierauf beschränkt.

Dampfspaltverfahren werden im kommerziellen Maßstab nahezu ausschließlich in Rohrreaktoren durchgeführt, in denen einzelne Reaktionsrohre (in Form von Rohrschlangen, sogenannten Coils) oder Gruppen von entsprechenden Reaktionsrohren auch bei unterschiedlichen Spaltbedingungen betrieben werden können. Unter gleichen oder vergleichbaren Spaltbedingungen betriebene Reaktionsrohre oder Gruppen von Reaktionsrohren, gegebenenfalls aber auch unter einheitlichen Spaltbedingungen betriebene Rohrreaktoren insgesamt, werden auch als "Spaltöfen" bezeichnet. Eine Anlage zum Dampfspalten (auch als "Olefinanlage" bezeichnet) kann einen oder mehrere Spaltöfen aufweisen.

Einem Spaltofen wird ein sogenannter "Ofeneinsatz" zugeführt und in diesem zumindest teilweise umgesetzt. Als Ofeneinsatz eignet sich eine Vielzahl von Kohlenwasserstoffen und Kohlenwasserstoffgemischen von Ethan bis Gasöl bis zu einem Siedepunkt von typischerweise 600 °C. Ein Ofe neinsatz kann aus einem sogenannten "Frischeinsatz" bestehen, also aus einem Einsatz, der anlagenextern bereitgestellt und beispielsweise aus einer oder mehreren Erdölfraktionen, Erdgas und/oder Erdgaskondensaten gewonnen wird. Ein Ofeneinsatz kann auch aus einem oder mehreren sogenannten "Recycleströmen" bestehen, also Strömen, die in der Anlage selbst erzeugt und in einen entsprechenden Spaltofen zurückgeführt werden. Ein Ofeneinsatz kann auch aus einem Gemisch eines oder mehrerer Frischeinsätze mit einem oder mehreren Recycleströmen bestehen.

Einem oder mehreren Spaltöfen wird ein sogenanntes "Rohgas" entnommen und geeigneten Nachbehandlungsschritten unterworfen. Diese umfassen zunächst eine Aufbereitung des Rohgases, beispielsweise durch Quenchen, Kühlen und Trocknen, wodurch ein sogenanntes "Spaltgas" erhalten wird. Bisweilen wird auch das Rohgas als Spaltgas bezeichnet.

Gängige Verfahren umfassen insbesondere die Trennung des Spaltgases in eine Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (jedoch konventionsgemäß unter Umständen auch Wasserstoff, dann auch "C1minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt auch für die "C5-Fraktion" und die höheren Fraktionen. Mehrere solcher Fraktionen können auch verfahrens- und/oder bezeichnungsmäßig zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen.

Flüssige und gasförmige Ströme können im hier verwendeten Sprachgebrauch reich oder arm an einer oder mehreren Komponenten sein, wobei "reich" für einen Gehalt von wenigstens 90%, 95%, 99%, 99,5%, 99,9%, 99,99% oder 99,999% und "arm" für einen Gehalt von höchstens 10%, 5%, 1%, 0,1%, 0,01% oder 0,001% auf molarer, Gewichts- oder Volumenbasis stehen kann. Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen, 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält.

Ein flüssiger oder gasförmiger Strom ist von einem anderen flüssigen oder gasförmigen Strom (auch als Ausgangsstrom bezeichnet) "abgeleitet", wenn er zumindest einige in dem Ausgangsstrom enthaltene oder aus diesem erhaltene Komponenten aufweist. Ein in diesem Sinne abgeleiteter Strom kann aus dem Ausgangsstrom durch Abtrennen oder Abzweigen eines Teilstroms oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten werden.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Ein "Wärmetauscher" dient zur indirekten Übertragung von Wärme zwischen zumindest zwei z.B. im Gegenstrom zueinander geführten Strömen, beispielsweise einem wärmeren gasförmigen Druckstrom und einem oder mehreren kälteren flüssigen Strömen. Ein Wärmetauscher kann aus einem einzelnen oder mehreren parallel und/oder seriell verbundenen Wärmetauscherabschnitten gebildet sein, z.B. aus einem oder mehreren Plattenwärmetauscherblöcken. Ein Wärmetauscher weist "Passagen" auf, die als getrennte Fluidkanäle mit Wärmeaustauschflächen ausgebildet sind.

Ein "Flüssigkeitsabscheider" oder "Abscheidebehälter" ist ein Behälter, in dem aus einem gasförmigen Strom oder einem Zweiphasenstrom (der zum Teil flüssig und zum Teil gasförmig vorliegt), eine Flüssigkeit, sogenanntes Kondensat, abgeschieden wird. Das Kondensat kann aus dem Flüssigkeitsabscheider (typischerweise aus einem unteren Bereich, "Sumpf") zumindest teilweise flüssig abgezogen werden, ein gasförmig verbliebener Anteil kann dem Flüssigkeitsabscheider (typischerweise aus einem oberen Bereich, "Kopf") zumindest teilweise gasförmig entnommen werden.

Bei einer "Destillationssäule" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, bereitgestelltes Stoffgemisch (Fluid) zumindest teilweise aufzutrennen, also aus dem Stoffgemisch jeweils Reinstoffe oder Stoffgemische zu erzeugen, die gegenüber dem Stoffgemisch bezüglich zumindest einer Komponente angereichert bzw. abgereichert oder reich bzw. arm im oben erläuterten Sinne sind. Destillationssäulen sind aus dem Bereich der Trenntechnik hinlänglich bekannt. Typischerweise sind Destillationssäulen als zylindrische Metallbehälter ausgebildet, die mit Einbauten, beispielsweise Siebböden oder geordneten oder ungeordneten Packungen, ausgerüstet sind. Eine Destillationssäule zeichnet sich unter anderem dadurch aus, dass sich in ihrem unteren Bereich, auch als Sumpf bezeichnet, eine flüssige Fraktion abscheidet. Diese flüssige Fraktion, die hier als Sumpfflüssigkeit bezeichnet wird, wird in einer Destillationssäule mittels eines Sumpfverdampfers erwärmt, so dass kontinuierlich ein Teil der Sumpfflüssigkeit verdampft und in der Destillationssäule gasförmig aufsteigt. Eine Destillationssäule ist ferner typischerweise mit einem sogenannten Kopfkondensator versehen, in den zumindest ein Teil eines sich in einem oberen Bereich der Destillationssäule anreichernden Gasgemischs oder ein entsprechendes Reingas, hier als Kopfgas bezeichnet, eingespeist, zu einem Teil zu einem Kondensat verflüssigt und als flüssiger Rücklauf am Kopf der Destillationssäule aufgegeben wird. Ein Teil des aus dem Kopfgas erhaltenen Kondensats kann anderweitig verwendet werden.

Im Gegensatz zu einer Destillationssäule verfügt eine "Absorptionskolonne" typischerweise nicht über einen Sumpfverdampfer. Auch Absorptionskolonnen sind aus dem Bereich der Trenntechnik allgemein bekannt. Absorptionskolonnen werden zur Absorption im Phasengegenstrom verwendet und daher auch als Gegenstromkolonnen bezeichnet. Bei der Absorption im Gegenstrom strömt die abgebende Gasphase aufwärts durch eine Absorptionskolonne. Die aufnehmende Lösungsphase fließt, von oben aufgegeben und unten abgezogen, der Gasphase entgegen. Die Gasphase wird mit der Lösungsphase "gewaschen". In einer entsprechenden Absorptionskolonne sind ebenfalls typischerweise Einbauten vorgesehen, die für einen stufenweisen (Böden, Sprühzonen, rotierende Teller usw.) oder stetigen (regellose Schüttungen von Füllkörpern, Packungen usw.) Phasenkontakt sorgen. Am Kopf einer solchen Absorptionskolonne wird ein gasförmiges Fluid erhalten, das als "Kopfprodukt" aus dieser abgezogen werden kann. Im Sumpf der Absorptionskolonne scheidet sich eine Flüssigkeit ab, die als "Sumpfprodukt" abgezogen werden kann. Die Gasphase wird in der Absorptionskolonne bezüglich einer oder mehrerer Komponenten abgereichert, welche in das Sumpfprodukt übergehen. Auch die als "C2-Absorber" oder "Absorber" bezeichnete Einrichtung, die in herkömmlichen Anlagen bei der Trennung eines C2minus-Stroms eingesetzt wird, ist eine Absorptionskolonne. Diese weist die zuvor erläuterte Anzahl an Böden auf und wird bei den bereits erwähnten Bedingungen betrieben. Der Absorber ist zusätzlich zu einer (Haupt-)Destillationssäule vorhanden, in der Methan einerseits und C2-Kohlenwasserstoffe andererseits (vorwiegend Ethan, Ethylen und ggf. Acetylen) voneinander getrennt werden. Der C2-Absorber dient im Wesentlichen zur vorgelagerten Abtrennung eines Wasserstoff und Methan enthaltenden Gemischs.

Zur Auslegung und spezifischen Ausgestaltung von Destillationssäulen und Absorptionskolonnen sei auf einschlägige Lehrbücher verwiesen (siehe beispielsweise Sattler, K.: Thermische Trennverfahren: Grundlagen, Auslegung, Apparate, 3. Auflage 2001, Weinheim, Wiley-VCH).

### Vorteile der Erfindung

Die vorliegende Erfindung geht von einem insoweit bekannten Verfahren zur Trennung eines Kohlenwasserstoffgemischs aus, das in Wesentlichen Kohlenwasserstoffe mit zwei Kohlenstoffatomen sowie Methan aufweist und ferner Wasserstoff enthält. Wie zuvor erläutert, wird ein derartiges Kohlenwasserstoffgemisch auch als C2 minus-Fraktion, C2-Strom usw. bezeichnet. Ein derartiges Verfahren wird, wie erwähnt, unter Verwendung einer Destillationssäule durchgeführt, in der Methan und C2-Kohlenwasserstoffe voneinander getrennt werden. In dem erfindungsgemäßen Verfahren wird dabei auch ein wasserstoffreicher Strom gewonnen.

Bei einem derartigen Verfahren wird Fluid des Kohlenwasserstoffgemischs auf einem ersten Druckniveau stufenweise von einem ersten Temperaturniveau über zwei oder mehrere Zwischentemperaturniveaus auf ein zweites Temperaturniveau abgekühlt. Auf jedem der Zwischentemperaturniveaus werden dabei Kondensate aus dem Fluid abgeschieden. Diese werden hier als "erste Kondensate" bezeichnet.

Wird im Rahmen dieser Anmeldung angegeben, dass "Fluid eines Stroms" oder "Fluid eines Kohlenwasserstoffgemischs" in irgendeiner Weise behandelt wird, sei hierunter verstanden, dass beispielsweise das gesamte Fluid, ein von einem Ausgangsfluid abgeleitetes Fluid oder ein Teilstrom eines Stroms, der aus einem entsprechenden Fluid gebildet wurde, verwendet wird. Insbesondere werden in dem zuvor erläuterten Verfahrensschritt jeweils ein Kondensat und ein gasförmig verbleibender Anteil aus einem entsprechenden Fluid gebildet. Der gasförmig verbleibende Anteil wird dabei jeweils auf ein nächsttieferes Temperaturniveau (ein Zwischentemperaturniveau oder letztlich das zweite Temperaturniveau) abgekühlt.

Fluid des Kohlenwasserstoffgemischs, das auf dem zweiten Temperaturniveau gasförmig verbleibt, also das Fluid des C2minus-Kohlenwasserstoffgemischs, das nicht in Form der ersten Kondensate abgeschieden wird, wird auf dem ersten Druckniveau in einen C2-Absorber eingespeist, dem am Kopf ein flüssiger Rücklauf aufgegeben wird. Aus dem Sumpf des C2-Absorbers wird ein Kondensat (hier als "zweites Kondensat" bezeichnet) und am Kopf des C2-Absorbers ein gasförmiger Kopfstrom abgezogen. Letzterer enthält überwiegend Methan und Wasserstoff. Auch hier wird also eine Trennung in ein Kondensat und in einen gasförmig verbleibenden Anteil vorgenommen.

Fluid des gasförmigen Kopfstroms vom Kopf des C2-Absorbers wird auf ein drittes Temperaturniveau abgekühlt und ebenfalls auf dem ersten Druckniveau in einen

Wasserstoffabscheider überführt. Bei dem Wasserstoffabscheider handelt es sich um einen weiteren Flüssigkeitsabscheider, der jedoch bei einer nochmals geringeren Temperatur, hier beispielsweise einer Temperatur von -150 °C betrieben wird. Bei dem genannten Druck von 34 bis 35 bar und der erwähnten Temperatur scheidet sich in dem Wasserstoffabscheider eine flüssige, methanreiche Fraktion am Boden ab. Es verbleibt ein gasförmiger, wasserstoffreicher Strom am Kopf, der eines der Produkte des Verfahrens darstellt.

Erfindungsgemäß ist vorgesehen, als den am Kopf des C2-Absorbers aufgegebenen Rücklauf Fluid eines Kondensats, das in dem Wasserstoffabscheider erhalten wird und im Rahmen dieser Anmeldung als "drittes Kondensat" bezeichnet wird, zu verwenden.

Die vorliegende Erfindung unterscheidet sich damit unter anderem dadurch vom Stand der Technik, das als Rücklauf in dem erwähnten C2-Absorber das methanreiche dritte Kondensat vom Sumpf des Wasserstoffabscheiders verwendet wird und nicht ein methanreicher flüssiger Strom, der unter Verwendung eines Kopfkondensators in der verwendeten Destillationssäule erhalten wird. Im Gegensatz zu dem bekannten Verfahren ermöglicht die vorliegende Erfindung damit den Verzicht auf eine Pumpe zur Überführung eines entsprechenden flüssigen Stroms aus der Destillationssäule beziehungsweise von deren Kopf zur Aufgabestelle an dem C2-Absorber. Ein ggf. vorhandener Druckunterschied zwischen dem Wasserstoffabscheider und dem C2-Absorber wird dabei durch Schwerkraftwirkung überwunden werden, indem der Wasserstoffabscheider in ausreichender Höhe geodätisch oberhalb des C2-Absorbers angeordnet wird. Das dritte Kondensat aus dem Wasserstoffabscheider fließt daher ausschließlich aufgrund der Schwerkraftwirkung zum C2-Absorber ab und überwindet damit den Druckunterschied.

Der Einsatz der erfindungsgemäßen Lösung verändert die Stoff- und Wärmebilanz eines entsprechenden Systems praktisch nicht und zieht keine weitere Beeinflussung des Gesamtverfahrens nach sich. Die Erfindung ermöglicht eine Reduktion der Investitionskosten und eine Vereinfachung des Anlagenbetriebs. Insbesondere betrifft dies das Anfahren und den Standby der Reservepumpe. Insgesamt ergibt sich eine Erhöhung der Anlagenverfügbarkeit: Pumpen und andere Apparate und Maschinen, auf die im Rahmen der vorliegenden Erfindung teilweise verzichtet werden kann, unterliegen höheren Beanspruchungen und Verschleiß. Sie sind dadurch störungsanfälliger als Komponenten ohne bewegliche Teile und fallen daher häufiger aus bzw. müssen gewartet werden. Durch Störungen und planmäßige Wartungen müssen bisweilen größere Anlagenteile bzw. -bereiche stillgelegt werden, so dass ggf. die Verfügbarkeit der Anlage insgesamt nicht mehr gegeben ist.

Im Rahmen der vorliegenden Erfindung kann eine herkömmlicherweise vorgesehene sogenannte "kalte Pumpe" für flüssiges Methan vollständig entfallen. Hierdurch erhöht sich die Verfügbarkeit und vereinfacht sich die Betreibbarkeit einer Olefinanlage, in die die erfindungsgemäße Trenneinrichtung integriert ist. Ferner reduzieren sich die Investitionskosten durch ein eingespartes Pumpenpaar, es ergibt sich eine vereinfachte und reduzierte Regelung und eventuell eine Verkürzung eines betroffenen Kolonnenabschnitts durch eine reduzierte Hold Up-Anforderung: Bekanntermaßen dürfen die beispielsweise für flüssiges Methan verwendeten Pumpen nicht trockenlaufen, so dass stets eine minimale Menge zu pumpender Flüssigkeit gefordert wird. Diese Menge, die typischerweise für vier bis fünf Minuten ausreichen muss, wird als "Hold Up" bezeichnet. Bei Ventilen ist der Hold Up geringer bzw. entfällt, weil diese bei einem sogenannten Gasdurchbruch nicht beschädigt werden.

In dem erfindungsgemäßen Verfahren wird also das als Rücklauf verwendete Fluid des dritten Kondensats ausschließlich durch Schwerkraftwirkung aus dem Wasserstoffabscheider in den C2-Absorber überführt. Eine Überführung "ausschließlich durch Schwerkraftwirkung" erfolgt dabei ohne Verwendung einer Pumpe, was die zuvor erläuterten Vorteile nach sich zieht. Insbesondere kann ein entsprechender Wasserstoffabscheider in den Kopf des verwendeten C2-Absorbers integriert werden, beispielsweise in Form eines Flüssigkeitsverschlussbodens. In diesem Fall kann eine Anpassung der verwendeten Regelung erfolgen. Eine Füllstandsregelung für einen entsprechenden Wasserstoffabscheider wird in diesem Fall durch eine Temperaturregelung ersetzt.

Erfindungsgemäß werden in dem Verfahren Fluid der ersten Kondensate und Fluid des zweiten Kondensats von dem ersten Druckniveau auf ein zweites Druckniveau unterhalb des ersten Druckniveaus entspannt und in die Destillationssäule eingespeist, die auf dem zweiten Druckniveau betrieben wird, wobei in der Destillationssäule zumindest ein flüssiger Strom, der im Wesentlichen aus Kohlenwasserstoffen mit zwei Kohlenstoffatomen besteht, und ein flüssiger Strom, der im Wesentlichen aus Methan besteht, gewonnen und aus der Destillationssäule abgezogen werden. Weil der flüssige Strom, der im Wesentlichen aus Methan besteht, nicht mittels einer Pumpe druckbeaufschlagt werden muss, um als Rücklauf auf den C2-Absorber aufgegeben zu werden, erweist sich das erfindungsgemäße Verfahren hier als besonders vorteilhaft.

Wie erläutert, wird die Destillationssäule im Rahmen des erfindungsgemäßen Verfahrens auf einem zweiten, tieferen Druckniveau betrieben als der C2-Absorber oder der Wasserstoffabscheider. Daher wäre es ohne eine entsprechende Pumpe nicht möglich, Fluid vom Kopf der Destillationssäule auf den C2-Absorber zu führen. Wie bereits eingangs erwähnt, erfolgt die Abkühlung des Einsatzstroms, der Betrieb des C2-Absorbers und der Betrieb des Wasserstoffabscheiders beispielsweise bei ca. 35 bar, allgemeiner bei 30 bis 40 bar (dem ersten Druckniveau), die Destillationssäule wird hingegen bei einem geringeren Druck betrieben, nämlich bei ca. 28 bis 34 oder 30 bis 32 bar (dem zweiten Druckniveau), wobei das zweite Druckniveau immer unterhalb des ersten Druckniveaus liegt.

Vorteilhafterweise wird dabei eine Menge des als Rücklauf verwendeten Fluids des dritten Kondensats derart eingestellt, dass es einer Menge des aus der Destillationssäule abgezogenen flüssigen Stroms, der im Wesentlichen aus Methan besteht, entspricht. Die Anlage kann daher ohne Einschränkungen weiter betrieben werden; die Kenngrößen ändern sich nicht.

Vorteilhafterweise wird Fluid des aus der Destillationssäule abgezogenen flüssigen Stroms zumindest zum Abkühlen des Fluids des Kohlenwasserstoffgemischs von dem ersten Temperaturniveau über die Zwischentemperaturniveaus auf das zweite Temperaturniveau verwendet. Dies ermöglicht eine effiziente Abkühlung entsprechenden Fluids.

Vorteilhafterweise wird ferner das Fluid des gasförmigen, wasserstoffreichen Stroms aus dem Wasserstoffabscheider ebenfalls zum Abkühlen des Fluids des Kohlenwasserstoffgemischs von dem ersten Temperaturniveau über die Zwischentemperaturniveaus auf das zweite Temperaturniveau und zum Abkühlen des Fluids des gasförmigen Kopfstroms vom Kopf des C2-Absorbers auf das dritte Temperaturniveau verwendet.

Das erfindungsgemäße Verfahren eignet sich in besonderer Weise zur Trennung eines Kohlenwasserstoffgemischs, das aus einem mittels eines Dampfspaltprozesses gewonnenen Spaltgas erhalten wird.

Eine entsprechende Trenneinrichtung ist ebenfalls Gegenstand der Erfindung. Die Trenneinrichtung ist zur Trennung eines im Wesentlichen aus Kohlenwasserstoffen mit zwei Kohlenstoffen sowie Methan und Wasserstoff bestehenden Kohlenwasserstoffgemischs eingerichtet und umfasst eine Destillationssäule, einen C2-Absorber und einen Wasserstoffabscheider. Zu den weiteren Bestandteilen einer entsprechenden Anlage sei auf die obigen Erläuterungen verwiesen.

Die Erfindung und Ausführungsformen der Erfindung werden unter Bezugnahme auf die beigefügte Zeichnung näher erläutert.

### Kurze Beschreibung der Zeichnungen

Figur 1 zeigt ein Verfahren zur Erzeugung von Kohlenwasserstoffen in Form eines schematischen Ablaufplans.
Figur 2 zeigt eine Trenneinrichtung zur Trennung eines Kohlenwasserstoffgemischs gemäß dem Stand der Technik.
Figur 3 zeigt eine Trenneinrichtung zur Trennung eines Kohlenwasserstoffgemischs gemäß einer Ausführungsform der Erfindung.

### Ausführliche Beschreibung der Zeichnungen

In den Figuren sind einander entsprechende Elemente mit identischen Bezugszeichen angegeben und werden der Übersichtlichkeit halber nicht wiederholt erläutert.

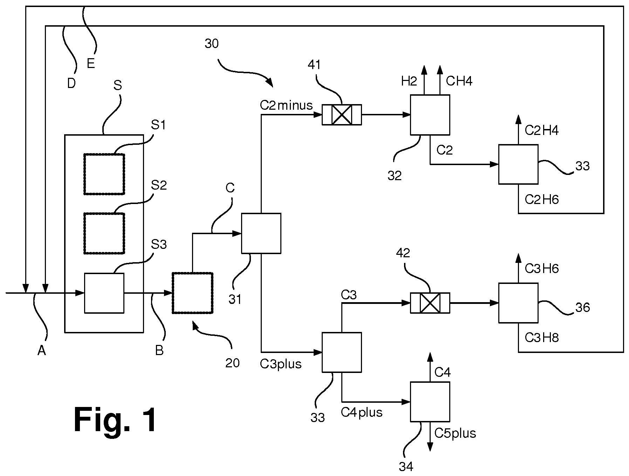

In Figur 1 ist der Ablauf eines Verfahrens zur Erzeugung von Kohlenwasserstoffen gemäß dem Stand der Technik in Form eines schematischen Ablaufplans dargestellt. Es ist ein Dampfspaltprozess S vorgesehen, der unter Verwendung eines oder mehrerer Spaltöfen S1 bis S3 durchgeführt werden kann. Nachfolgend wird nur der Betrieb des Spaltofens S3 erläutert, die weiteren Spaltöfen S1 und S2 können in entsprechender Weise arbeiten oder wegfallen.

Der Spaltofen S3 wird mit einem Strom A als Ofeneinsatz beschickt, bei dem es sich zumindest teilweise um einen sogenannten Frischeinsatz handeln kann, der aus anlagenexternen Quellen zur Verfügung gestellt wird, und zu einem Teil um einen sogenannten Recyclestrom, der in dem Verfahren selbst gewonnen wird, wie unten erläutert. Auch die anderen Spaltöfen S1 und S2 können mit entsprechenden Strömen beschickt werden. Unterschiedliche Ströme können auch in unterschiedliche Spaltöfen S1 bis S3 eingespeist werden, ein Strom kann auf mehrere Spaltöfen S1 bis S3 aufgeteilt werden oder mehrere Teilströme können zu einem Sammelstrom vereinigt werden, der beispielsweise als Strom A einem der Spaltöfen S1 bis S3 zugeführt wird.

Durch Dampfspalten in dem Dampfspaltprozess S wird ein Rohgasstrom B erhalten, der bisweilen bereits an dieser Stelle als Spaltgasstrom bezeichnet wird. Der Rohgasstrom B wird in einer Reihe nicht dargestellter Aufbereitungsstufen eines Aufbereitungsprozesses 20 aufbereitet, beispielsweise einem sogenannten Ölquench unterzogen, vorfraktioniert, verdichtet, weiter gekühlt und getrocknet.

Der entsprechend behandelte Strom B, das eigentliche Spaltgas C, wird anschließend einem Trennprozess 30 unterworfen. In diesem wird eine Anzahl von Fraktionen gewonnen, die hier, wie eingangs erläutert, entsprechend der Kohlenstoffanzahl der überwiegend enthaltenen Kohlenwasserstoffe bezeichnet werden. Der in der Figur 1 dargestellte Trennprozess 30 arbeitet nach dem Prinzip "Deethanizer First".

Dem Fachmann sind, beispielsweise aus dem eingangs erwähnten Artikel "Ethylene" in Ullmann's Encyclopedia of Industrial Chemistry, mehrere Verfahrensalternativen bekannt, die sich insbesondere in der Aufbereitung des Spaltgases C und/oder dem verwendeten Trennprozess unterscheiden. Es sei ausdrücklich betont, dass sich die Erfindung beispielsweise auch bei "Demethanizer First"-Verfahren einsetzen lässt.

In dem Trennprozess 30 wird dabei aus dem Spaltgas C zunächst in einer Trenneinrichtung 31 eine C2minus-Fraktion gasförmig abgetrennt, die überwiegend Methan, Ethan, Ethylen und Acetylen und insbesondere auch noch Wasserstoff enthalten kann. Die C2minus-Fraktion wird insgesamt einem Hydrotreatmentprozess 41 unterzogen, um enthaltenes Acetylen zu Ethylen umzusetzen. Anschließend werden aus der C2minus-Fraktion in einer C2minus-Trenneinrichtung 32 Methan CH4 und Wasserstoff H2 nacheinander oder gemeinsam abgetrennt und beispielsweise als Brenngas verwendet. Die vorliegende Erfindung betrifft insbesondere die Trenneinrichtung 32, die in schematischer Teildarstellung auch in den nachfolgenden Figuren veranschaulicht wird.

Es verbleibt eine C2-Fraktion, die in einer C2-Trenneinrichtung 32 in Ethylen C2H4 und Ethan C2H6 aufgetrennt wird. Letzteres kann auch als Recyclestrom D in einem oder mehreren Spaltöfen S1 bis S3 erneut dem Dampfspaltprozess S unterworfen werden. Im dargestellten Beispiel werden die Recycleströme D und E zu dem Strom A zugegeben. Die Recycleströme D und E und der Strom A können auch in unterschiedliche Spaltöfen S1 bis S3 geführt werden.

In der Trenneinrichtung 31 verbleibt eine flüssige C3plus-Fraktion die in eine Trenneinrichtung 33 (den sogenannten Depropanizer) überführt wird. In der Trenneinrichtung 33 wird aus der C3plus-Fraktion eine C3-Fraktion abgetrennt und einem Hydrotreatmentprozess 42 unterzogen, um in der C3-Fraktion enthaltenes Methylacatylen zu Propylen umzusetzen. Anschließend wird die C3-Fraktion in einer C3- Trenneinrichtung 34 in Propen C3H6 und Propan C3H8 aufgetrennt. Letzteres kann als Recyclestrom E in einem oder mehreren Spaltöfen S1 bis S3, separat oder mit anderen Strömen, erneut dem Dampfspaltprozess S unterworfen werden.

In der Trenneinrichtung 33 verbleibt eine flüssige C4plus-Fraktion, die in eine vierte Trenneinrichtung 34 (den sogenannten Debutanizer) überführt wird. In der Trenneinrichtung 34 wird aus der C4plus-Fraktion eine C4-Fraktion gasförmig abgetrennt. Es verbleibt eine flüssige C5plus-Fraktion.

Es versteht sich, dass sämtliche der dargestellten Fraktionen auch geeigneten Nachbehandlungsschritten unterworfen werden können. Beispielsweise kann aus der C4-Fraktion 1,3-Butadien abgetrennt werden. Es können ferner zusätzliche Recycleströme verwendet werden, die analog zu den Recycleströmen D und E dem Dampfspaltprozess S unterworfen werden können.

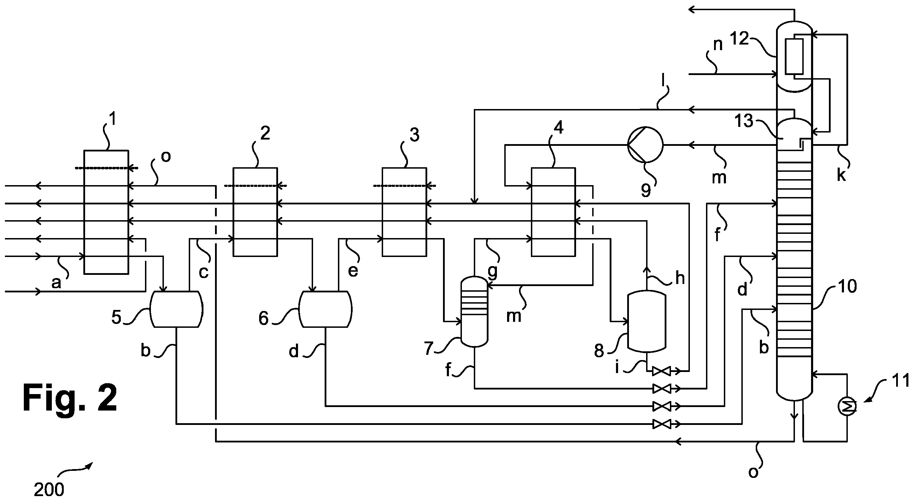

Figur 2 zeigt eine Trenneinrichtung zur Trennung eines Kohlenwasserstoffgemischs gemäß dem Stand der Technik. Die Trenneinrichtung ist insgesamt mit 200 bezeichnet und zur Trennung eines Kohlenwasserstoffgemischs, das im Wesentlichen aus Kohlenwasserstoffen mit zwei Kohlenstoffatomen, Methan und Wasserstoff besteht (also einer C2minus-Fraktion) eingerichtet. Die C2minus-Fraktion wird der Trenneinrichtung 200 in Form eines Stroms a zugeführt.

Die Trenneinrichtung 200 umfasst einen ersten Wärmetauscher 1, einen zweiten Wärmetauscher 2, einen dritten Wärmetauscher 3 und einen vierten Wärmetauscher 4. Der Strom a wird zunächst durch den ersten Wärmetauscher 1 geführt und in diesem abgekühlt. Er wird anschließend in einen ersten Flüssigkeitsabscheider 5 eingespeist. Die Abkühlung im den ersten Wärmetauscher 1 erfolgt derart, dass sich im den ersten Flüssigkeitsabscheider 5 ein flüssiges Kondensat abscheidet. Dieses wird am Boden des ersten Flüssigkeitsabscheiders 5 als Strom b abgezogen. Die weitere Verwendung des Stroms b wird unten erläutert.

Ein in dem ersten Flüssigkeitsabscheider 5 gasförmig verbliebener Anteil des Stroms a wird als Strom c durch den zweiten Wärmetauscher 2 geführt und anschließend in einen zweiten Flüssigkeitsabscheider 6 eingespeist. Auch in diesem scheidet sich ein flüssiges Kondensat am Boden ab und wird in Form des Stroms d abgezogen. Ein noch immer gasförmig verbliebener Anteil des Stroms c wird als Strom e in dem dritten Wärmetauscher 3 abgekühlt und in einen C2-Absorber 7 eingespeist. Auch in dem C2-Absorber 7 scheidet sich im Sumpf ein flüssiges Kondensat ab, das als Strom f abgezogen wird. Zusätzlich wird dem C2-Absorber 7 am Kopf ein Strom m aufgegeben, dessen Herkunft unten erläutert wird. Ein vom Kopf des C2-Absorbers 7 abgezogenes Kopfgas wird in Form des Stroms g durch den vierten Wärmetauscher 4 geführt und anschließend in einen Wasserstoffabscheider 8 eingespeist.

Am Boden des Wasserstoffabscheiders 8 scheidet sich ein methanreiches Kondensat ab, das als Strom i abgezogen und in umgekehrter Reihenfolge und Richtung durch den vierten bis ersten Wärmetauscher 4 bis 1 geführt wird. In dieser Reihenfolge und Richtung wird auch ein Strom h eines wasserstoffreichen Kopfgases vom Kopf des Wasserstoffabscheiders 8 durch den vierten bis ersten Wärmetauscher 4 bis 1 geführt. Die Darstellung ist hier insbesondere insoweit stark vereinfacht, dass Querverbindungen zwischen den Leitungen, in denen bestimmte Ströme, beispielsweise die Ströme i und h geführt werden, nicht veranschaulicht sind. Beispielsweise kann eine derartige Querverbindung erlauben, einen bestimmten Anteil des Stroms h dem Strom i zuzumischen. Auch sind Leitungen nicht gezeigt, die im Wesentlichen zum Anfahren einer entsprechenden Anlage verwendet werden. Beispielsweise kann in einer Trenneinrichtung 200 vorgesehen sein, während des Anfahrens den Strom m nicht am Kopf des C2-Absorbers 7 aufzugeben, sondern entsprechend dem Strom i durch den vierten bis ersten Wärmetauscher 4 bis 1 zu führen.

Die Trenneinrichtung 200 umfasst ferner eine Destillationssäule 10, die mit einem nicht näher erläuterten Sumpfverdampfer 11 betrieben wird, dessen Wärmetauscher beispielsweise mit einem aus anderen Anlagenteilen stammenden Propylenstrom betrieben wird. Die Destillationssäule 10 umfasst ferner einen Kopfkondensator 12, dessen Betrieb unten erläutert wird.

Aufgrund der sukzessiven Abkühlung der Ströme a, c und e weisen die entsprechend erhaltenen Kondensate, die in Form der Ströme b, d und f gewonnen werden, unterschiedliche Gehalte an Kohlenwasserstoffen mit zwei Kohlenstoffatomen und Methan auf. Insbesondere weist der Strom f einen höheren Methangehalt als der Strom d und der Strom d einen höheren Methangehalt als der Strom b auf.

Die Ströme b, d und f werden daher in unterschiedlichen Höhen in die Destillationssäule 10 eingespeist, die hierzu geeignete Aufgabeeinrichtungen zwischen den hier stark schematisiert dargestellten Böden aufweist.

Vom Kopf der Destillationssäule 10 wird ein gasförmiger Strom k abgezogen und in einem Kondensationsraum des Kopfkondensators 12 verflüssigt. Der verflüssigte Strom wird in einem Bereich 13 am Kopf der Destillationssäule 10 in eine flüssige und eine gasförmige Phase getrennt. Die gasförmige Phase geht in den Gasraum der Destillationssäule 10 über und vereinigt sich am Kopf der Destillationssäule 10 mit weiterem Kopfgas. Aus dem Bereich 13 kann ein gasförmiger Strom I abgezogen und mit dem zuvor erläuterten Strom i stromab des vierten Wärmetauschers 4 vereinigt werden. Der Strom I enthält überwiegend Methan.

Ein entsprechender flüssiger methanreicher Strom m kann ebenfalls aus dem Bereich 13 abgezogen werden. Der Strom m wird mittels einer Pumpe 9 (sogenannte kalte Pumpe) dem warmen Ende des vierten Wärmetauschers 4 zugeführt und in dem vierten Wärmetauscher 4 abgekühlt. Er wird anschließend, wie bereits erwähnt, am Kopf des C2-Absorbers 7 aufgegeben. Der Kopfkondensator 12 der Destillationssäule 10 kann mit einem Strom n aus anderen Anlagenteilen als Kältemittel beschickt werden. Beispielsweise handelt es sich hierbei um einen Ethylenstrom.

Im Sumpf der Destillationssäule 10 scheidet sich ein flüssiges Kondensat ab, das im Wesentlichen aus Kohlenwasserstoffen mit zwei Kohlenstoffatomen besteht (es handelt sich also um eine sogenannte C2-Fraktion). Das Kondensat wird in Form des Stroms o abgezogen, in dem ersten Wärmetauscher 1 erwärmt und beispielsweise anschließend einer weiteren Trenneinrichtung zugeführt. Wie anfangs erwähnt, sind Pumpen für tiefkalte Medien, beispielsweise flüssiges Methan, aufwendig zu betreiben und bedürfen erhöhter Aufmerksamkeit im Betrieb. Dies betrifft in der in Figur 2 dargestellten Trenneinrichtung 200 insbesondere die Methanpumpe 9.

In der Trenneinrichtung 200, die in Figur 2 dargestellt ist, erfolgt eine Regelung der am Kopf des C2-Absorbers eingespeisten Menge des Stroms m auf Grundlage eines Füllstands eines entsprechenden Kondensats in dem Bereich 13 der Destillationssäule 10. Ein Druck des verdampften Kältemittels (Strom n) in dem Kopfkondensator 12 wird auf Grundlage einer in der Destillationssäule 10 gemessenen Temperatur geregelt. Die Menge des in Form des Stroms n eingespeisten Kältemittels wird auf Grundlage eines Füllstands in dem Kopfkondensator 12 geregelt.

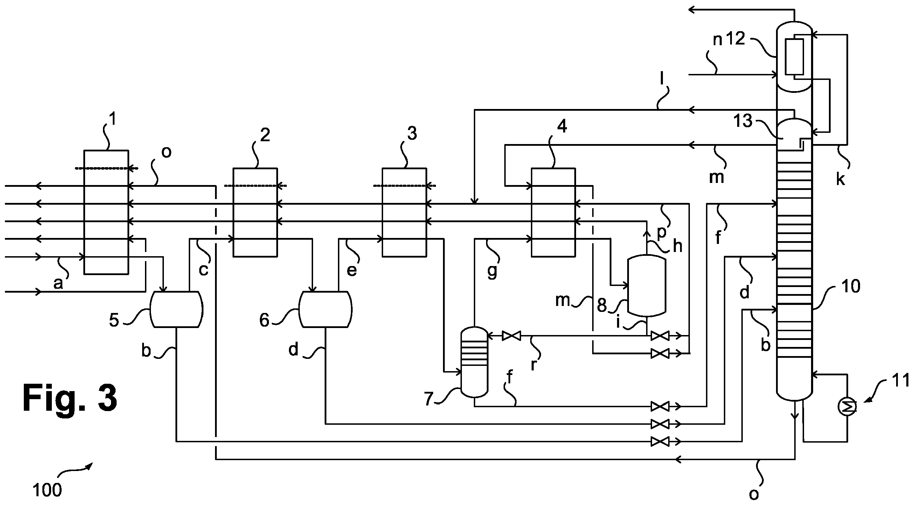

Figur 3 zeigt eine Trenneinrichtung 100 gemäß einer Ausführungsform der Erfindung. Die Trenneinrichtung 100 umfasst die wesentlichen Komponenten der in Figur 2 dargestellten Trenneinrichtung 200. Diese werden nicht erneut erläutert.

Abweichend zu der in Figur 2 dargestellten Trenneinrichtung 100 wurde hier jedoch auf die Pumpe 9 verzichtet. Ferner wird der Strom m nicht am Kopf des C2-Absorbers 7 aufgegeben sondern mit dem Strom i aus dem Sumpf des Methanabscheiders 8 zu einem Strom p vereinigt. Der Strom p wird, statt wie zuvor nur der Strom i, in der zuvor erläuterten Reihenfolge durch den vierten bis ersten Wärmetauscher 4 bis 1 geführt.

Vor der Vereinigung mit dem Strom m wird jedoch insbesondere ein Teilstrom r des Stroms i abgezweigt, der anstelle des Stroms m (vergleiche Trenneinrichtung 200 der Figur 2) am Kopf des C2-Absorbers 7 aufgegeben wird. Wird dabei, wie in Figur 3 ebenfalls schematisch veranschaulicht, zumindest die Aufgabestelle für den Strom I in dem C2-Absorber 7 unterhalb einer Entnahmestelle für den Strom i aus dem Wasserstoffabscheider 8 angeordnet, kann in dem Wasserstoffabscheider 8 anfallendes Kondensat unter Restriktion durch ein entsprechendes Ventil (ohne Bezeichnung) schwerkraftgetrieben als Rücklauf auf den C2-Absorber geführt werden. Die Menge des in dem in Form des Stroms r am Kopf des C2-Absorbers aufgegebenen Kondensats aus dem Wasserstoffabscheider 8 entspricht vorteilhafterweise der aus der Destillationssäule 10 in Form des Stroms m abgezogenen Methanmenge und wird durch diese zur Kälteerzeugung in dem vierten Wärmetauscher 4 ersetzt. Weil zur entsprechenden Speisung des vierten Wärmetauschers 4 nur ein geringes Druckniveau erforderlich ist, kann auf die Methanpumpe 9 verzichtet werden.

## Patentansprüche

1. Verfahren zur Trennung eines Wasserstoff enthaltenden Kohlenwasserstoffgemischs (C2minus), das neben dem Wasserstoff im Wesentlichen Kohlenwasserstoffe mit zwei Kohlenstoffatomen und Methan enthält, unter Verwendung einer Destillationssäule (10), bei dem
- Fluid (a, c, e) des Kohlenwasserstoffgemischs (C2minus) auf einem ersten Druckniveau stufenweise von einem ersten Temperaturniveau über zwei oder mehrere Zwischentemperaturniveaus auf ein zweites Temperaturniveau abgekühlt wird, wobei auf jedem der Zwischentemperaturniveaus erste Kondensate (b, d) aus dem Fluid (a, c, e) abgeschieden werden,
- Fluid (e) des Kohlenwasserstoffgemischs (C2minus), das auf dem zweiten Temperaturniveau gasförmig verbleibt, auf dem ersten Druckniveau in einen C2-Absorber (7) eingespeist wird, dem am Kopf ein flüssiger Rücklauf (r) aufgegeben wird, wobei aus dem Sumpf des C2-Absorbers (7) ein zweites Kondensat (f) und am Kopf des C2-Absorbers (7) ein überwiegend Methan und Wasserstoff enthaltender, gasförmiger Kopfstrom (g) abgezogen werden,
- Fluid des gasförmigen Kopfstroms (g) vom Kopf des C2-Absorbers (7) auf ein drittes Temperaturniveau abgekühlt und auf dem ersten Druckniveau in einen Wasserstoffabscheider (8) überführt wird, in dem aus dem Fluid des gasförmigen Kopfstroms (g) unter Verbleib eines gasförmigen, wasserstoffreichen Stroms (h) ein methanreiches drittes Kondensat (i) abgeschieden wird, und
- Fluid der ersten Kondensate (b, d) und Fluid des zweiten Kondensats (f) von dem ersten Druckniveau auf ein zweites Druckniveau unterhalb des ersten Druckniveaus entspannt und in die Destillationssäule (10) eingespeist wird, die auf dem zweiten Druckniveau betrieben wird, wobei in der Destillationssäule (10) zumindest ein flüssiger Strom (o), der im Wesentlichen aus Kohlenwasserstoffen mit zwei Kohlenstoffatomen besteht, und ein flüssiger Strom (m), der im Wesentlichen aus Methan besteht, gewonnen und aus der Destillationssäule (10) abgezogen werden,
**dadurch gekennzeichnet, dass** der am Kopf des C2-Absorbers (7) aufgegebene Rücklauf (r) aus Fluid des dritten Kondensats (i) gebildet wird, das in dem Wasserstoffabscheider (8) aus dem Fluid des gasförmigen Kopfstroms (g) vom Kopf des C2-Absorbers (7) abgeschieden und ausschließlich durch Schwerkraftwirkung aus dem Wasserstoffabscheider (8) in den C2-Absorber (7) überführt wird.

2. Verfahren nach Anspruch 1, bei dem eine Menge des als Rücklauf (r) verwendeten Fluids des dritten Kondensats (i) derart eingestellt wird, dass sie einer Menge des aus der Destillationssäule (10) abgezogenen flüssigen Stroms (m), der im Wesentlichen aus Methan besteht, entspricht.

3. Verfahren nach Anspruch 1 oder 2, bei dem Fluid des flüssigen Stroms (m), der aus der Destillationssäule (10) abgezogen wird, zumindest zum Abkühlen des Fluids (a, c, e) des Kohlenwasserstoffgemischs (C2minus) von dem ersten Temperaturniveau über die Zwischentemperaturniveaus auf das zweite Temperaturniveau verwendet wird.

4. Verfahren nach einem der vorstehenden Ansprüche, bei dem Fluid des gasförmigen, wasserstoffreichen Stroms (h) aus dem Wasserstoffabscheider (8) zum Abkühlen des Fluids (a, c, e) des Kohlenwasserstoffgemischs (C2minus) von dem ersten Temperaturniveau über die Zwischentemperaturniveaus auf das zweite Temperaturniveau und des Fluids des gasförmigen Kopfstroms (g) vom Kopf des C2-Absorbers (7) auf das dritte Temperaturniveau verwendet wird.

5. Verfahren nach einem der vorstehenden Ansprüche, das zur Trennung eines Kohlenwasserstoffgemischs (C2minus) eingesetzt wird, das aus einem mittels eines Dampfspaltprozesses (50) gewonnenen Spaltgas erhalten wird.

6. Trenneinrichtung (100), die zur Trennung eines Wasserstoff enthaltenden Kohlenwasserstoffgemischs (C2minus), das neben Wasserstoff im Wesentlichen Kohlenwasserstoffe mit zwei Kohlenstoffatomen und Methan enthält, eingerichtet ist und zumindest eine Destillationssäule (10), einen C2-Absorber (7) sowie einen Wasserstoffabscheider (8) sowie Mittel aufweist, die dafür eingerichtet sind,
- Fluid (a, c, e) des Kohlenwasserstoffgemischs (C2minus) auf einem ersten Druckniveau stufenweise von einem ersten Temperaturniveau über zwei oder mehrere Zwischentemperaturniveaus auf ein zweites Temperaturniveau abzukühlen sowie auf jedem der Zwischentemperaturniveaus erste Kondensate (b, d) aus dem Fluid (a, c, e) abzuscheiden,
- Fluid (e) des Kohlenwasserstoffgemischs (C2minus), das auf dem zweiten Temperaturniveau gasförmig verbleibt, auf dem ersten Druckniveau in den C2-Absorber (7) einzuspeisen, dem C2-Absorber (7) am Kopf einen flüssigen Rücklauf (r) aufzugeben, und aus dem Sumpf des C2-Absorbers (7) ein zweites Kondensat (f) und am Kopf des C2-Absorbers (7) einen überwiegend Methan und Wasserstoff enthaltenden, gasförmigen Kopfstrom (g) abzuziehen,
- Fluid des gasförmigen Kopfstroms (g) vom Kopf des C2-Absorbers (7) auf ein drittes Temperaturniveau abzukühlen und auf dem ersten Druckniveau in den Wasserstoffabscheider (8) zu überführen und in diesem aus dem Fluid des gasförmigen Kopfstroms (g) unter Verbleib eines gasförmigen, wasserstoffreichen Stroms (h) ein methanreiches drittes Kondensat (i) abzuscheiden, und
- Fluid der ersten Kondensate (b, d) und Fluid des zweiten Kondensats (f) von dem ersten Druckniveau auf ein zweites Druckniveau unterhalb des ersten Druckniveaus zu entspannen und in die Destillationssäule (10) einzuspeisen, die Destillationssäule auf dem zweiten Druckniveau zu betreiben, und in der Destillationssäule (10) zumindest einen flüssigen Strom (o), der im Wesentlichen aus Kohlenwasserstoffen mit zwei Kohlenstoffatomen besteht, und einen flüssigen Strom (m), der im Wesentlichen aus Methan besteht, zu gewinnen und aus der Destillationssäule (10) abzuziehen,
**dadurch gekennzeichnet, dass** Mittel vorgesehen sind, die dafür eingerichtet sind, den am Kopf des C2-Absorbers (7) aufgegebenen Rücklauf (r) aus Fluid des dritten Kondensats (i) zu bilden, das in dem Wasserstoffabscheider (8) aus dem Fluid des gasförmigen Kopfstroms (g) vom Kopf des C2-Absorbers (7) abgeschieden wird, wobei der Wasserstoffabscheider geodätisch oberhalb des C2-Absorbers angeordnet ist, so dass das dritte Kondensat aus dem Wasserstoffabscheider ausschließlich aufgrund der Wirkung der Schwerkraft zum C2-Absorber abfließt.

7. Trenneinrichtung (100) nach Anspruch 6, die zur Durchführung eines Verfahrens nach einem der Ansprüche 1 bis 5 eingerichtet ist.

8. Olefinanlage, die zur Durchführung eines Dampfspaltprozesses (50) unter Verwendung wenigstens eines Spaltofens (51 - 53) eingerichtet ist, mit Mitteln, die dafür eingerichtet sind, aus Fluid eines Spaltgases (C) des wenigstens einen Dampfspaltprozesses (50) ein Kohlenwasserstoffgemisch (C2minus), das im Wesentlichen aus Kohlenwasserstoffen mit zwei Kohlenstoffatomen sowie Methan und Wasserstoff besteht zu gewinnen, **gekennzeichnet durch** zumindest eine Trenneinrichtung (100) nach Anspruch 6 oder 7, die zur Trennung des Kohlenwasserstoffgemischs (C2minus) eingerichtet ist.

## Claims

1. Process for separating a hydrogen-comprising hydrocarbon mixture (C2minus) comprising in addition to the hydrogen essentially hydrocarbons having two carbon atoms and methane using a distillation column (10) where
- fluid (a, c, e) of the hydrocarbon mixture (C2minus) at a first pressure level is cooled stepwise from a first temperature level via two or more intermediate temperature levels to a second temperature level, wherein at each of the intermediate temperature levels first condensates (b, d) are separated from the fluid (a, c, e),
- fluid (e) of the hydrocarbon mixture (C2minus) which at the second temperature level remains gaseous is fed at the first pressure level into a C2 absorber (7) to the top of which a liquid reflux (r) is applied, wherein from the bottom of the C2 absorber (7) a second condensate (f), and at the top of the C2 absorber (7) a predominantly methane- and hydrogen-comprising gasesous tops stream (g) are withdrawn,
- fluid of the gaseous tops stream (g) from the top of the C2 absorber (7) is cooled to a third temperature level and at the first pressure level is transferred into a hydrogen separator (8) in which from the fluid of the gaseous tops stream (g), to leave behind a gaseous hydrogen-rich stream (h), a methane-rich third condensate (i) is separated and
- fluid of the first condensates (b, d) and fluid of the second condensate (f) are decompressed from the first pressure level to a second pressure level below the first pressure level and fed into the distillation column (10) which is operated at the second pressure level, wherein in the distillation column (10) at least a liquid stream (o) which consists essentially of hydrocarbons having two carbon atoms and a liquid stream (m) which consists essentially of methane are derived and are withdrawn from the distillation column (10),
**characterized in that** the reflux (r) applied at the top of the C2 absorber (7) is formed from fluid of the third condensate (i) which is separated in the hydrogen separator (8) from the fluid of the gaseous tops stream (g) from the top of the C2 absorber (7) and transferred exclusively by the action of gravity from the hydrogen separator (8) into the C2 absorber (7).

2. Process according to Claim 1, where an amount of the fluid of the third condensate (i) used as reflux (r) is adjusted such that it corresponds to an amount of the liquid stream (m) withdrawn from the distillation column (10) which consists essentially of methane.

3. Process according to Claim 1 or 2, where the fluid of the liquid stream (m) which is withdrawn from the distillation column (1.0) is used at least for cooling the fluid (a, c, e) of the hydrocarbon mixture (C2minus) from the first temperature level via the intermediate temperature levels to the second temperature level.

4. Process according to any of the preceding claims, where the fluid of the gaseous, hydrogen-rich stream (h) from the hydrogen separator (8) is used for cooling the fluid (a, c, e) of the hydrocarbon mixture (C2minus) from the first temperature level via the intermediate temperature levels to the second temperature level and the fluid of the gaseous tops stream (g) from the top of the C2 absorber (7) to the third temperature level.

5. Process according to any of the preceding claims, used for separating a hydrocarbon mixture (C2minus) obtained from a cracking gas derived from a steamcracking process (50).

6. Separating apparatus (100), which is set up for separating a hydrogen-comprising hydrocarbon mixture (C2minus) comprising in addition to hydrogen essentially hydrocarbons having two carbon atoms and methane, and which comprises at least a distillation column (10), a C2 absorber (7) and a hydrogen separator (8) and means set up for
- cooling fluid (a, c, e) of the hydrocarbon mixture (C2minus) at a first pressure level stepwise from a first temperature level via two or more intermediate temperature levels to a second temperature level and separating at each of the intermediate temperature levels first condensates (b, d) from the fluid (a, c, e),
- feeding at the first pressure level into the C2 absorber (7) fluid (e) of the hydrocarbon mixture (C2minus) which at the second temperature level remains gaseous, applying to the top of the C2 absorber (7) liquid reflux (r) and withdrawing from the bottom of the C2 absorber (7) a second condensate (f), and at the top of the C2 absorber (7) a predominantly methane- and hydrogen-comprising gasesous tops stream (g),
- cooling fluid of the gaseous tops stream (g) from the top of the C2 absorber (7) to a third temperature level and at the first pressure level transferring it into the hydrogen separator (8) and therein separating from the fluid of the gaseous tops stream (g), to leave behind a gaseous hydrogen-rich stream (h), a methane-rich third condensate (i) and
- decompressing fluid of the first condensates (b, d) and fluid of the second condensate (f) from the first pressure level to a second pressure level below the first pressure level and feeding it into the distillation column (10), operating the distillation column at the second pressure level, and in the distillation column (10) deriving at least a liquid stream (o) which consists essentially of hydrocarbons having two carbon atoms and a liquid stream (m) which consists essentially of methane and withdrawing these from the distillation column (10),
**characterized in that** means are provided which are set up for forming the reflux (r) applied at the top of the C2 absorber (7) from fluid of the third condensate (i) which is separated in the hydrogen separator (8) from the fluid of the gaseous tops stream (g) from the top of the C2 absorber (7), wherein the hydrogen separator is arranged geodetically above the C2 absorber so that the third condensate from the hydrogen separator drains to the C2 absorber exclusively due to the action of gravity.

7. Separating apparatus (100) according to Claim 6, set up for performing a process according to any of Claims 1 to 5.

8. Olefin plant set up for performing a steamcracking process (50) using at least one cracking furnace (51-53) comprising means set up for deriving from fluid of a cracking gas (C) from the at least one steamcracking process (50) a hydrocarbon mixture (C2minus) which consists essentially of hydrocarbons having two carbon atoms and methane and hydrogen, **characterized by** at least one separating apparatus (100) according to Claim 6 or 7, set up for separating the hydrocarbon mixture (C2minus).

## Revendications

1. Procédé de séparation d'un mélange d'hydrocarbures contenant de l'hydrogène (C2minus) qui, outre l'hydrogène, contient essentiellement des hydrocarbures à deux atomes de carbone et du méthane, utilisant une colonne de distillation (10), selon lequel
- un fluide (a, c, e) du mélange d'hydrocarbures (C2minus) est refroidi par paliers à un premier niveau de pression d'un premier niveau de température à un deuxième niveau de température par le biais de deux niveaux de température intermédiaires ou plus, des premiers condensats (b, d) étant séparés du fluide (a, c, e) à chacun des niveaux de température intermédiaires,
- un fluide (e) du mélange d'hydrocarbures (C2minus), qui reste sous forme gazeuse au deuxième niveau de température, est introduit au premier niveau de pression dans un absorbeur de C2 (7), à la tête duquel un reflux liquide (r) est introduit, un deuxième condensat (f) étant soutiré du fond de l'absorbeur de C2 (7) et un courant de tête gazeux (g) contenant principalement du méthane et de l'hydrogène à la tête de l'absorbeur de C2 (7),
- un fluide du courant de tête gazeux (g) issu de la tête de l'absorbeur de C2 (7) est refroidi à un troisième niveau de température et transféré au premier niveau de pression dans un séparateur d'hydrogène (8), dans lequel un troisième condensat (i) riche en méthane est séparé à partir du fluide du courant de tête gazeux (g) en laissant un courant gazeux riche en hydrogène (h), et
- un fluide des premiers condensats (b, d) et un fluide du deuxième condensat (f) sont détendus du premier niveau de pression à un deuxième niveau de pression inférieur au premier niveau de pression et introduits dans la colonne de distillation (10), qui est exploitée au deuxième niveau de pression, au moins un courant liquide (o), qui est essentiellement constitué d'hydrocarbures à deux atomes de carbone, et un courant liquide (m), qui est essentiellement constitué de méthane, étant obtenus dans la colonne de distillation (10) et soutirés de la colonne de distillation (10), **caractérisé en ce que** le reflux (r) introduit à la tête de l'absorbeur de C2 (7) est formé par un fluide du troisième condensat (i), qui est séparé dans le séparateur d'hydrogène (8) à partir du fluide du courant de tête gazeux (g) issu de la tête de l'absorbeur de C2 (7) et qui est transféré exclusivement sous l'effet de la force de pesanteur depuis le séparateur d'hydrogène (8) dans l'absorbeur de C2 (7).

2. Procédé selon la revendication 1, selon lequel une quantité du fluide du troisième condensat (i) utilisé en tant que reflux (r) est ajustée de manière à correspondre à une quantité du courant liquide (m) soutiré de la colonne de distillation (10), qui est essentiellement constitué de méthane.

3. Procédé selon la revendication 1 ou 2, selon lequel un fluide du courant liquide (m), qui est soutiré de la colonne de distillation (10), est utilisé au moins pour le refroidissement du fluide (a, c, e) du mélange d'hydrocarbures (C2minus) du premier niveau de température au deuxième niveau de température par le biais des niveaux de température intermédiaires.

4. Procédé selon l'une quelconque des revendications précédentes, selon lequel un fluide du courant gazeux riche en hydrogène (h) issu du séparateur d'hydrogène (8) est utilisé pour le refroidissement du fluide (a, c, e) du mélange d'hydrogène (C2minus) du premier niveau de température au deuxième niveau de température par le biais des niveaux de température intermédiaires et du fluide du courant de tête gazeux (g) issu de la tête de l'absorbeur de C2 (7) au troisième niveau de température.

5. Procédé selon l'une quelconque des revendications précédentes, qui est utilisé pour la séparation d'un mélange d'hydrocarbures (C2minus) qui est obtenu à partir d'un gaz de craquage obtenu par un procédé de vapocraquage (50).

6. Dispositif de séparation (100), qui est conçu pour la séparation d'un mélange d'hydrocarbures contenant de l'hydrogène (C2minus) qui, outre l'hydrogène, contient essentiellement des hydrocarbures à deux atomes de carbone et du méthane, et comprend au moins une colonne de distillation (10), un absorbeur de C2 (7) et un séparateur d'hydrogène (8), ainsi que des moyens conçus pour
- refroidir par paliers un fluide (a, c, e) du mélange d'hydrocarbures (C2minus) à un premier niveau de pression d'un premier niveau de température à un deuxième niveau de température par le biais de deux niveaux de température intermédiaires ou plus, et pour séparer des premiers condensats (b, d) du fluide (a, c, e) à chacun des niveaux de température intermédiaires,
- introduire un fluide (e) du mélange d'hydrocarbures (C2minus), qui reste sous forme gazeuse au deuxième niveau de température, au premier niveau de pression dans l'absorbeur de C2 (7), introduire à la tête de l'absorbeur de C2 (7) un reflux liquide (r), et soutirer un deuxième condensat (f) du fond de l'absorbeur de C2 (7) et un courant de tête gazeux (g) contenant principalement du méthane et de l'hydrogène à la tête de l'absorbeur de C2 (7),
- refroidir un fluide du courant de tête gazeux (g) issu de la tête de l'absorbeur de C2 (7) à un troisième niveau de température et le transférer au premier niveau de pression dans le séparateur d'hydrogène (8), et séparer dans celui-ci un troisième condensat (i) riche en méthane à partir du fluide du courant de tête gazeux (g) en laissant un courant gazeux riche en hydrogène (h), et
- détendre un fluide des premiers condensats (b, d) et un fluide du deuxième condensat (f) du premier niveau de pression à un deuxième niveau de pression inférieur au premier niveau de pression et les introduire dans la colonne de distillation (10), exploiter la colonne de distillation au deuxième niveau de pression, et obtenir au moins un courant liquide (o), qui est essentiellement constitué d'hydrocarbures à deux atomes de carbone, et un courant liquide (m), qui est essentiellement constitué de méthane, dans la colonne de distillation (10) et les soutirer de la colonne de distillation (10), **caractérisé en ce que** des moyens sont prévus, qui sont conçus pour former le reflux (r) introduit à la tête de l'absorbeur de C2 (7) à partir d'un fluide du troisième condensat (i), qui est séparé dans le séparateur d'hydrogène (8) à partir du fluide du courant de tête gazeux (g) issu de la tête de l'absorbeur de C2 (7), le séparateur d'hydrogène étant agencé géodésiquement au-dessus de l'absorbeur de C2, de sorte que le troisième condensat s'écoule exclusivement sous l'effet de la force de pesanteur depuis le séparateur d'hydrogène dans l'absorbeur de C2.

7. Dispositif de séparation (100) selon la revendication 6, qui est conçu pour la réalisation d'un procédé selon l'une quelconque des revendications 1 à 5.

8. Unité de production d'oléfines, qui est conçue pour la réalisation d'un procédé de vapocraquage (50) utilisant au moins un four de craquage (51 à 53), qui comprend des moyens conçus pour obtenir à partir d'un fluide d'un gaz de craquage (C) dudit au moins un procédé de vapocraquage (50) un mélange d'hydrocarbures (C2minus), qui est essentiellement constitué d'hydrocarbures à deux atomes de carbone et de méthane et d'hydrogène, **caractérisé par** au moins un dispositif de séparation (100) selon la revendication 6 ou 7, qui est conçu pour la séparation du mélange d'hydrocarbures (C2minus).
